# EUROPEAN PATENT APPLICATION

(11) **EP 1 918 371 A1**
(43) Date of publication of application: **07.05.2008**
(21) Application number: 06781181.0
(22) Date of filing: 18.07.2006
(51) Int. Cl.: C12N 15/00, A23L 1/30, A61K 38/00, C12N 9/16, C12N 15/09, C12P 9/00, C12P 19/44

(54) **NOVEL PHOSPHOLIPID PROCESSING AGENT**

(30) Priority: 19.07.2005 JP 2005208913; 03.03.2006 JP 2006057141
(71) Applicant: Asahi Kasei Pharma Corporation, Tokyo 101-8481 (JP)
(72) Inventor: KOGA, Shinji, Tokyo 1008440 (JP); IMAMURA, Shigeyuki, Tokyo 1008440 (JP)
(74) Representative: Forstmeyer, Dietmar
(86) International application number: PCT/JP2006/314160
(87) International publication number: WO 2007/010892

(57) **Abstract**

Problems: It is an object of the present invention to provide a novel phospholipase B (PLB) having less hydrolytic activity on phosphatidylinositol (PI) among diacyl form of the phospholipid. Also, it is an object of the invention to provide a method of efficiently manufacturing PI and glyceryl phosphoryl choline (GPC) from a phospholipid mixture by using a substrate specificity of an enzyme.

Means to Solve the Problems: Provided is a phospholipid processing agent containing an enzyme, which acts effectively on a phospholipid other than PI, such as phosphatidyl choline (lecithin), and having less PLB activity on PI.

SEQUENCE LISTING

<110> Asahi Kasei Pharma Corporation

<120> New reagent containing Phospholipase B

<130> F106074

<140>

<141>

<150> JP2005-208913

<151> 2005-7-19

<150> JP2006-057141

<151> 2006-3-3

<160> 3

<170> PatentIn Ver. 2.1

<210> 1

<211> 549

<212> PRT

<213> Candida

<220>

<223>Inventor : Imamura, Shigeyuki

<400> 1

Met Glu Leu Ala Leu Ala Leu Leu Leu Ile Ala Ser Val Ala Ala Ala

1               5                   10                  15

Pro Thr Ala Thr Leu Ala Asn Gly Asp Thr Ile Thr Gly Leu Asn Ala

            20                  25                  30

Ile Ile Asn Glu Ala Phe Leu Gly Ile Pro Phe Ala Glu Pro Pro Val

        35                  40                  45
Gly Asn Leu Arg Phe Lys Asp Pro Val Pro Tyr Ser Gly Ser Leu Asp
    50                  55                  60
Gly Gln Lys Phe Thr Leu Tyr Gly Pro Leu Cys Met Gln Gln Asn Pro
65                  70                  75                  80
Glu Gly Thr Tyr Glu Glu Asn Leu Pro Lys Ala Ala Leu Asp Leu Val
                85                  90                  95
Met Gln Ser Lys Val Phe Glu Ala Val Leu Pro Leu Ser Glu Asp Cys
            100                 105                 110
Leu Thr Ile Asn Val Val Arg Pro Pro Gly Thr Lys Ala Gly Ala Asn
        115                 120                 125
Leu Pro Val Met Leu Trp Ile Phe Gly Gly Gly Phe Glu Val Gly Gly
    130                 135                 140
Thr Ser Thr Phe Pro Pro Ala Gln Met Ile Thr Lys Ser Ile Ala Met
145                 150                 155                 160
Gly Lys Pro Ile Ile His Val Ser Val Asn Tyr Arg Val Ser Ser Trp
                165                 170                 175
Gly Phe Leu Ala Gly Asp Glu Ile Lys Ala Glu Gly Ser Ala Asn Ala
            180                 185                 190
Gly Leu Lys Asp Gln Arg Leu Gly Met Gln Trp Val Ala Asp Asn Ile
        195                 200                 205
Ala Ala Phe Gly Gly Asp Pro Thr Lys Val Thr Ile Phe Gly Glu Leu
    210                 215                 220
Ala Gly Ser Met Ser Val Met Cys His Ile Leu Trp Asn Asp Gly Asp

225                 230                 235                 240
Asn Thr Tyr Lys Gly Lys Pro Leu Phe Arg Ala Gly Ile Met Gln Leu
                245                 250                 255
Gly Ala Met Val Pro Leu Asp Ala Val Asp Gly Ile Tyr Gly Asn Glu
            260                 265                 270
Ile Phe Asp Leu Leu Ala Ser Asn Ala Gly Cys Gly Ser Ala Ser Asp
        275                 280                 285
Lys Leu Ala Cys Leu Arg Gly Val Leu Ser Asp Thr Leu Glu Asp Ala
    290                 295                 300
Thr Asn Asn Thr Pro Gly Phe Leu Ala Tyr Ser Ser Leu Arg Leu Leu
305                 310                 315                 320
Tyr Leu Pro Arg Pro Asp Gly Val Asn Ile Thr Asp Asp Met Tyr Ala
                325                 330                 335
Leu Val Arg Glu Gly Lys Tyr Ala Asn Ile Pro Val Ile Ile Gly Asp
            340                 345                 350
Gln Asn Asp Glu Gly Thr Phe Phe Gly Thr Leu Leu Leu Asn Val Thr
        355                 360                 365
Thr Asp Ala Gln Ala Arg Glu Tyr Phe Lys Gln Leu Phe Val His Ala
    370                 375                 380
Ser Asp Ala Glu Ile Asp Thr Leu Met Thr Ala Tyr Pro Gly Asp Ile
385                 390                 395                 400
Thr Gln Gly Leu Pro Phe Asp Thr Gly Ile Leu Asn Ala Leu Thr Pro
                405                 410                 415
Gln Phe Lys Arg Ile Leu Ala Val Leu Gly Asp Leu Gly Phe Thr Leu

            420                 425                 430

Ala Arg Arg Tyr Phe Leu Asn His Tyr Thr Gly Gly Thr Lys Tyr Ser

        435                 440                 445

Phe Leu Leu Lys Gln Leu Leu Gly Leu Pro Val Leu Gly Thr Phe His

    450                 455                 460

Ser Asn Asp Ile Val Phe Gln Asp Tyr Leu Leu Gly Ser Gly Ser Leu

465                 470                 475                 480

Ile Tyr Asn Asn Ala Phe Ile Ala Phe Ala Thr Asp Leu Asp Pro Asn

                485                 490                 495

Thr Ala Gly Leu Leu Val Lys Trp Pro Glu Tyr Thr Ser Ser Leu Gln

            500                 505                 510

Leu Gly Asn Asn Leu Met Met Ile Asn Ala Leu Gly Leu Tyr Thr Gly

        515                 520                 525

Lys Asp Asn Phe Arg Thr Ala Gly Tyr Asp Ala Leu Phe Ser Asn Pro

    530                 535                 540

Pro Leu Phe Phe Val

545

<210> 2

<211> 549

<212> PRT

<213> Candida

<220>

<223>Inventor : Imamura, Shigeyuki

<400> 2

Met Lys Leu Ala Leu Ala Leu Leu Leu Ile Ala Ser Val Ala Ala Ala
1               5                   10                  15
Pro Thr Ala Lys Leu Ala Asn Gly Asp Thr Ile Thr Gly Leu Asn Ala
            20                  25                  30
Ile Ile Asn Glu Ala Phe Leu Gly Ile Pro Phe Ala Glu Pro Pro Val
        35                  40                  45
Gly Asn Leu Arg Phe Lys Asp Pro Val Pro Tyr Ser Gly Ser Leu Asn
    50                  55                  60
Gly Gln Lys Phe Thr Leu Tyr Gly Pro Leu Cys Met Gln Gln Asn Pro
65                  70                  75                  80
Glu Gly Thr Phe Glu Glu Asn Leu Gly Lys Thr Ala Leu Asp Leu Val
                85                  90                  95
Met Gln Ser Lys Val Phe Gln Ala Val Leu Pro Gln Ser Glu Asp Cys
            100                 105                 110
Leu Thr Ile Asn Val Val Arg Pro Pro Gly Thr Lys Ala Gly Ala Asn
        115                 120                 125
Leu Pro Val Met Leu Trp Ile Phe Gly Gly Gly Phe Glu Ile Gly Ser
    130                 135                 140
Pro Thr Ile Phe Pro Pro Ala Gln Met Val Thr Lys Ser Val Leu Met
145                 150                 155                 160
Gly Lys Pro Ile Ile His Val Ala Val Asn Tyr Arg Val Ala Ser Trp
                165                 170                 175
Gly Phe Leu Ala Gly Asp Asp Ile Lys Ala Glu Gly Ser Gly Asn Ala
            180                 185                 190

Gly Leu Lys Asp Gln Arg Leu Gly Met Gln Trp Val Ala Asp Asn Ile
        195                 200                 205
Ala Gly Phe Gly Gly Asp Pro Ser Lys Val Thr Ile Phe Gly Glu Leu
    210                 215                 220
Ala Gly Ser Met Ser Val Leu Cys His Leu Ile Trp Asn Asp Gly Asp
225                 230                 235                 240
Asn Thr Tyr Lys Gly Lys Pro Leu Phe Arg Ala Gly Ile Met Gln Leu
                245                 250                 255
Gly Ala Met Val Pro Leu Asp Pro Val Asp Gly Thr Tyr Gly Asn Glu
            260                 265                 270
Ile Tyr Asp Leu Phe Val Ser Ser Ala Gly Cys Gly Ser Ala Ser Asp
        275                 280                 285
Lys Leu Ala Cys Leu Arg Ser Ala Leu Ser Asp Thr Leu Leu Asp Ala
    290                 295                 300
Thr Asn Asn Thr Pro Gly Phe Leu Ala Tyr Ser Ser Leu Arg Leu Leu
305                 310                 315                 320
Tyr Leu Pro Arg Pro Asp Gly Lys Asn Ile Thr Asp Asp Met Tyr Lys
                325                 330                 335
Leu Val Arg Asp Gly Lys Tyr Ala Ser Val Pro Val Ile Ile Gly Asp
            340                 345                 350
Gln Asn Asp Glu Gly Thr Ile Phe Gly Leu Leu Leu Leu Asn Val Thr
        355                 360                 365
Thr Asn Ala Gln Ala Arg Ala Tyr Phe Lys Gln Leu Phe Ile His Ala
    370                 375                 380

Ser Asp Ala Glu Ile Asp Thr Leu Met Ala Ala Tyr Pro Gln Asp Ile
385                 390                 395                 400
Thr Gln Gly Leu Pro Phe Asp Thr Gly Ile Phe Asn Ala Ile Thr Pro
                405                 410                 415
Gln Phe Lys Arg Ile Leu Ala Val Leu Gly Asp Leu Ala Phe Ile His
            420                 425                 430
Ala Arg Arg Tyr Phe Leu Asn His Phe Gln Gly Gly Thr Lys Tyr Ser
        435                 440                 445
Phe Leu Leu Lys Gln Leu Leu Gly Leu Pro Ile Met Gly Thr Phe His
    450                 455                 460
Ala Asn Asp Ile Val Trp Gln Asp Tyr Leu Leu Gly Ser Gly Ser Val
465                 470                 475                 480
Ile Tyr Asn Asn Ala Phe Ile Ala Phe Ala Thr Asp Leu Asp Pro Asn
                485                 490                 495
Thr Ala Gly Leu Leu Val Asn Trp Pro Lys Tyr Thr Ser Ser Leu Gln
            500                 505                 510
Leu Gly Asn Asn Leu Met Met Ile Asn Ala Leu Gly Leu Tyr Thr Gly
        515                 520                 525
Lys Asp Asn Phe Arg Thr Ala Gly Tyr Asp Ala Leu Met Thr Asn Pro
    530                 535                 540
Leu Leu Phe Phe Val
545
<210> 3
<211> 1650

<212> DNA

<213> Candida

<220>

<223>Inventor : Imamura, Shigeyuki

<400> 3

atggagctcg ctcttgcgct cctgctcatt gcctcggtgg ctgctgcccc caccgccacg 60

ctcgccaacg gcgacaccat caccggtctc aacgccatca tcaacgaggc gttcctcggc 120

attccctttg ccgagccgcc ggtgggcaac ctccgcttca aggaccccgt gccgtactcc 180

ggctcgctcg atggccagaa gttcacgctg tacggcccgc tgtgcatgca gcagaacccc 240

gagggcacct acgaggagaa cctccccaag gcagcgctcg acttggtgat gcagtccaag 300

gtgtttgagg cggtgctgcc gctgagcgag gactgtctca ccatcaacgt ggtgcggccg 360

ccgggcacca aggcgggtgc caacctcccg gtgatgctct ggatctttgg cggcgggttt 420

gaggtgggtg gcaccagcac cttccctccc gcccagatga tcaccaagag cattgccatg 480

ggcaagccca tcatccacgt gagcgtcaac taccgcgtgt cgtcgtgggg gttcttggct 540

ggcgacgaga tcaaggccga gggcagtgcc aacgccggtt tgaaggacca gcgcttgggc 600

atgcagtggg tggcggacaa cattgcggcg tttggcggcg acccgaccaa ggtgaccatc 660

tttggcgagc tggcgggcag catgtcggtc atgtgccaca ttctctggaa cgacggcgac 720

aacacgtaca agggcaagcc gctcttccgc gcgggcatca tgcagctggg ggccatggtg 780

ccgctggacg ccgtggacgg catctacggc aacgagatct ttgacctctt ggcgtcgaac 840

gcgggctgcg gcagcgccag cgacaagctt gcgtgcttgc gcggtgtgct gagcgacacg 900

ttggaggacg ccaccaacaa cacccctggg ttcttggcgt actcctcgtt gcggttgctg 960

tacctccccc ggcccgacgg cgtgaacatc accgacgaca tgtacgcctt ggtgcgcgag 1020

ggcaagtatg ccaacatccc tgtgatcatc ggcgaccaga acgacgaggg caccttcttt 1080

ggcaccctgc tgttgaacgt gaccacggat gcccaggccc gcgagtactt caagcagctg 1140

tttgtccacg ccagcgacgc ggagatcgac acgttgatga cggcgtaccc cggcgacatc 1200
acccagggcc tgccgttcga cacgggtatt ctcaacgccc tcaccccgca gttcaagaga 1260
atcctggcgg tgctcggcga ccttggcttt acgcttgctc gtcgctactt cctcaaccac 1320
tacaccggcg gcaccaagta ctcattcctc ctgaagcagc tcctgggctt gccggtgctc 1380
ggaacgttcc actccaacga cattgtcttc caggactact tgttgggcag cggctcgctc 1440
atctacaaca acgcgttcat tgcgtttgcc acggacttgg accccaacac cgcggggttg 1500
ttggtgaagt ggcccgagta caccagcagc ctgcagctgg gcaacaactt gatgatgatc 1560
aacgccttgg gcttgtacac cggcaaggac aacttccgca ccgccggcta cgacgcgttg 1620
ttctccaacc cgccgctgtt ctttgtgtaa 1650

## Description

### TECHNICAL FIELD

The present invention relates to a novel phospholipid processing agent having a high phospholipase B (PLB) activity and substrate specificity to phospholipid.

### BACKGROUND ART

Phospholipase is a generic name for enzymes that hydrolyze phospholipids. Phospholipid (glycerol phospholipid) has fatty acids, which are ester-linked to a hydroxyl group on an α-position and β-position of glycerol while it has choline, ethanolamine, inositol, or the like, which is linked to a hydroxyl group of the other α-position of the glycerol through a phosphate group.
An enzyme hydrolyzing a fatty acid ester bond at the α-position of the glycerol group of glycerol phospholipid is generally called phospholipase A1. An enzyme hydrolyzing a fatty acid ester group of the β-position of the glycerol group is generally called phospholipase A2. In addition, an enzyme having both a phospholipase A1 activity and a phospholipase A2 activity is generally called phospholipase B (PLB).
Further, phospholipid in which only one of α-position and β-position fatty acid acyl groups therein is removed from the phospholipid is generally called lysophospholipid. An enzyme that hydrolyzes a fatty acid ester bond which is remaining after acting with lysophospholipid generates the same decomposition product as that of the above Mentioned PLB. Therefore, such an enzyme can also be included in the family of PLBs.
On the other hand, an enzyme hydrolyzing the ester bond between a glycerol group and a phosphate group in phospholipid is generally called phospholipase C (PLC). In addition, an enzyme hydrolyzing the linkage of a phosphate group with choline, ethanolamine, or the like is generally called phospholipase D (PLD).
As described above, PLBs also have lysophospholipase activities. The PLBs are known to be present in animals, plants, mold of Penicillium, Escherichia coli, yeast, and the like. The action thereof on phospholipid that is a diacyl form is extremely weak while the action thereof on lysolecithin is strong. Thus, PLBs have never been useful in order to effectively hydrolyze common phospholipids. In addition, for those known PLBs, it have simply been reported that they can act on a wide variety of phospholipids, such as phosphatidyl choline, phosphatidyl ethanolamine, phosphatidylserine, and phosphatidylinositol (Non-Patent Documents 1 and 2).
Various reports have reported that phosphatidylinositols are contained in soybean lecithin and closely relate to signal transduction of the cell. In recent years, it has been reported that intake of phosphatidylinositol could decrease the triacylglycerol (TG) level, and increase the level of HDL-C (high density lipoprotein cholesterol) in blood (Non-Patent Document 3). The physiological functions of phosphatidylinositols are drawing attentions in the art, as well as the physiological derivatives thereof, such as lysophosphatidylinositol and glyceryl phosphoryl inositol.
In addition, there is also a report that lysophosphatidylinositols have antimold actions (Patent Document 1).
Attempts to obtain phosphatidylinositol in an enzyme specific manner by the action of PLC or PLD on phospholipid have been performed. However, the purity of the resulting phosphatidylinositol is low. Therefore, the more selective method for the production of phosphatidylinositol has been demanded in the art (Patent Document 2). Non-Patent Document 2 also describes that the reaction is terminated at a hydrolysis rate of about 30% if the PLC is allowed to act on any phospholipid other than phosphatidylinositol.
Patent Document 1: JP H06-256366 A
Patent Document 2: JP S62-048390 A
Non-Patent Document 1: Biochimica Biophysica Acta (1974) 369, 245-253
Non-Patent Document 2: Biochimica BiophysicaActa (1975) 403, 412-424
Non-Patent Document 3: Biochem. J (2004) 382, 441-449
Non-Patent Document 4: Jim W. Burgess et al., Journal of Lipid Research (2005) 46, 350-355

### DISCLOSURE OF THE INVENTION

### PROBLEM TO BE SOLVED BY THE INVENTION

It is an object of the present invention to provide a phospholipid processing agent having a high PLB activity and substrate specificity to phospholipid, and to a method of efficiently producing high-purity phosphatidylinositol and glyceryl phosphoryl choline.

### MEANS FOR SOLVING THE PROBLEM

As a result of intensive studies for solving the above-described problems, the present inventors have found that an enzyme derived from the genus Candida surprisingly has a high PLB activity to the diacyl form of the phospholipid and the enzyme unexpectedly has specificity to the phospholipid that the enzyme selectively hydrolyzes the phospholipids other than phosphatidylinositol, of soybean phospholipids, which is a mixture of phospholipids, and have found a method of efficiently manufacturing high-purity phosphatidylinositol and glyceryl phosphoryl choline utilizing the specificity. Finally, the present inventors have completed the present invention based on such findings. The enzyme derived from the genus Candida is commonly used for a lipase in food industries and productions of raw materials for pharmaceutical products. However, it has only been reported that the enzyme has a so-called "lipase activity" to act on neutral lipids. There is no report that the enzyme has the PLB activity.
That is, the present invention relates to the followings:
<1> A phospholipid processing agent, including: an enzyme derived from the genus Candida and having a phospholipase B (PLB) activity.
<2> A phospholipid processing agent, including: an enzyme having a phospholipase B (PLB) activity, which is virtually free from decomposition of only phosphatidylinositol in a phospholipid mixture.
<3> A phospholipid processing agent according to Item <1> or <2>, in which the enzyme having the phospholipase B (PLB) activity further has a lipase activity.
<4> The phospholipid processing agent according to any one of Items <1> to <3>, in which the enzyme having the PLB activity has the following physicochemical properties:
   (1) action: an action of hydrolyzing phospholipid into free fatty acid at a molar ratio of two and glyceryl phosphoryl choline at an equimolar ratio;
   (2) molecular weight: 53,000±3,000 (determined by SDS electrophoresis);
   (3) isoelectric point: pH 4.21±0.2;
   (4) optimal pH: about pH 5.5 to 6.5;
   (5) pH stability: about pH 5 to 9 (37°C, 90 Minute treatment); and
   (6) stability: 55°C (10 Minute treatment at pH 5).
   (7) substrate specificity: an activity ratio to phosphatidylinositol is 10% or less of that to phosphatidyl choline.
<5> A phospholipid processing agent including an enzyme obtained from a culture medium of Candida cylindracea.
<6> A phospholipid processing agent, which is obtained by the following steps of:
   (1) culturing Candida cylindracea;
   (2) condensing a culture medium of Candida cylindracea;
   (3) precipitating enzymes with an organic solvent;
   (4) purifying a crude enzyme solution obtained by the step (3) by hydrophobic chromatography; and
   (5) separating and purifying the enzyme obtained by the step (4) by ion-exchange chromatography.
<7> A phospholipid processing agent including at lest one enzyme selected from: an enzyme having an amino acid sequence of SEQ ID No. 1; an en enzyme having a PLB activity and 75% or more homology to the amino acid sequence of SEQ ID No. 1; and an enzyme having the PLB activity and having the amino acid sequence of SEQ ID No. 1 with one to several amino acids being deleted, substituted, or added.
<8> A phospholipid processing agent, including at lest one enzyme selected from: an enzyme having an amino acid sequence of SEQ ID No. 2; an en enzyme having a PLB activity and 75% or more homology to the amino acid sequence of SEQ ID No. 2; and an enzyme having the PLB activity and having the amino acid sequence of SEQ ID No. 2 with one to several amino acids being deleted, substituted, or added.
<9> A method of manufacturing phosphatidylinositol and glyceryl phosphoryl choline including allowing the phospholipid processing agent according to any one of Items 1 to 8 to act on a phospholipid mixture.
<10> A method of manufacturing phosphatidylinositol including allowing the phospholipid processing agent according to any one of Items 1 to 8 to act on a phospholipid mixture.
<11> A method of manufacturing phosphatidylinositol, including the steps of:
   (1) allowing the phospholipid processing agent according to any one of <1> to <8> to act on a phospholipid mixture;
   (2) extracting phosphatidylinositol using an organic solvent; and
   (3) precipitating and recovering phosphatidylinositol by a water-soluble or water-miscible alkyl carbonyl alkyl solvent treatment.
<12> The method of manufacturing phosphatidylinositol according to any one of Items <9> to <11>, in which the phospholipid mixture is derived from soybean.
<13> A phosphatidylinositol, which is obtained by the manufacturing method according to any one of Items <10> to <12>, and having a phosphatidylinositol purity of 50 mol% or more in total phospholipids.
<14> A method of manufacturing glyceryl phosphoryl choline including allowing the phospholipid processing agent according to any one of Items <1> to <8> to act on a phospholipid mixture.
<15> A method of manufacturing glyceryl phosphoryl choline, including the steps of:
   (1) allowing the phospholipid processing agent according to any one of <1> to <8> to act on a phospholipid mixture;
   (2) extracting and removing lipid components using a solvent containing an organic solvent and collecting the glyceryl phosphoryl choline in an aqueous layer; and
   (3) adsorbing the phospholipid processing agent acting in the step (1) on activated carbon and removing the phospholipid processing agent.
<16> The method of manufacturing the glyceryl phosphoryl choline according to item <9>, <14>, or <15>, in which the phospholipid mixture is derived from soybean.
<17> A glyceryl phosphoryl choline, which is obtained by the manufacturing method according to any one of Items <14> to <16>, and having a glyceryl phosphoryl choline purity of 55 wt% or more.
<18> Use of the phosphatidylinositol according to Item <13> for the production of high-purity glycerophosphoinositol.
<19> Use of the glyceryl phosphoryl choline according to Item <17> for the production of high-purity choline phospholipid.
<20> Food, medicine, or cosmetic, containing the phosphatidylinositol manufactured by the manufacturing method according to any one of Items <10> to <12>.
<21> Food, medicine, or cosmetic, containing glyceryl phosphoryl choline manufactured by the manufacturing method according to any one of Items <14> to <16>.
<22> A lysophosphatidylinositol obtained by allowing an enzyme having phospholipase A1 or phospholipase A2 activity to act on the phosphatidylinositol manufactured by the manufacturing method according to any one of Items <10> to <12>.
<23> A method of manufacturing lysophosphatidylinositol, including allowing an enzyme having phospholipase A1 or phospholipase A2 activity to act on the phosphatidylinositol manufactured by the manufacturing method according to any one of the Items <10> to <12>.
<24> Food, medicine, or cosmetic, containing the lysophosphatidylinositol manufactured by the manufacturing method according to Item <23>.
<25> A glyceryl phosphoryl inositol obtained by allowing an enzyme having phospholipase B activity which acts on phosphatidylinositol well to act on the phosphatidylinositol manufactured by the manufacturing method according to any one of Items <10> to <12>.
<26> A method of manufacturing glyceryl phosphoryl inositol, including allowing an enzyme having phospholipase B activity which acts on phosphatidylinositol well to act on the phosphatidylinositol manufactured by the manufacturing method according to any one of Items <10> to <12>.
<27> Food, medicine, or cosmetic, containing the glyceryl phosphoryl inositol manufactured by the manufacturing method according to Item <26>.
<28> Food, medicine, or cosmetic, containing two or more kinds selected from the group consisting of: the phosphatidylinositol manufactured by the manufacturing method according to Items <10> to <12>; the glyceryl phosphoryl choline manufactured by the manufacturing method according to Items <14> to <16>; the lysophosphatidylinositol manufactured by the manufacturing method according to Item <23>; and the glyceryl phosphoryl inositol manufactured by the manufacturing method according to Item <26>.

### EFFECTS OF THE INVENTION

The use of the phospholipid processing agent of the present invention enables producing high-purity phosphatidylinositol, lysophosphatidylinositol, glyceryl phosphoryl choline, glyceryl phosphoryl inositol, and the like, which are useful as functional phospholipids or functional phospholipid raw materials.

### BEST MODE FOR CARRYING OUT THE INVENTION

Hereinafter, constituents and preferred embodiments of the present invention will be further described in detail.

### 1. Phospholipase B (PLB) as phospholipid processing agent

In this specification, the term "PLB" means an enzyme for carrying out hydrolysis of fatty acid esters at α-position and β-position of phospholipid, fatty acid ester synthesis, and fatty acid ester exchange. The PLBs of the present invention include any enzyme that acts on lysophospholipids and hydrolyze the remaining fatty acid ester bonds. Further, the term "phospholipids processing" means reactions, such as the hydrolysis of phospholipid, the fatty aid ester synthesis, the fatty acid ester exchange, or the like. Preferably, it means the hydrolysis or fatty acid ester synthesis, more preferably the hydrolysis. In another preferable mode, it means the fatty acid ester synthesis. Further, the processing agents of the present invention include not only a PLB itself, but also a PLB added with enzyme stabilizers, such as sugars, and pH buffers. In addition, the phospholipid processing agents can be provided in a manner similar to the conventional enzymes in dry powder, liquid, or the like.
According to the present invention, there is provided an phospholipid processing agent having PLB activity which substantially does not decompose only phosphatidylinositol in a phospholipid mixture while other components of the mixture are decomposed.
The phrase "substantially does not decompose only phosphatidylinositol", in terms of phospholipid processing agent, means that the activity ratio (relative activity) of phosphatidylinositol in a phospholipid mixture, which is a substrate to phosphatidyl choline, is 10% or less as the upper limit. It means that the activity ratio thereof is preferably 7% or less, more preferably 5% or less, particularly preferably 3% or less, most preferably 1% or less. It also means that the activity ratio thereof is 0.01% or more as a lower limit, more preferably 0.1% or more. Further, it means that the activity ratio (relative activity) of phospholipid other than phosphatidylinositol and phosphatidyl choline (for example, phosphatidyl ethanolamine, phosphatidylserine, or phosphatidic acid (PA)) to phosphatidyl choline is 15% or more as the lower limit. It means that the activity ratio thereof is preferably 20% or more, more preferably 25% or more, particularly preferably 30% or more, most preferably 40% or more. It means that the activity ratio thereof is 200% or less as an upper limit, preferably 150% or less, more preferably 100% or less.

In addition, PLB as a phospholipid processing agent of the present invention preferably has following properties:
1) Action: the action of hydrolyzing phospholipid such as lecithin into free fatty acid at a molar ratio of two and glyceryl phosphoryl choline at an equimolar ratio; and the action of acting on triglyceride to hydrolyze it into free fatty acid at a molar ratio of three and glycerin at an equimolar ratio.
2) molecular weight: 53,000±3,000 (determined by SDS electrophoresis).
3) Isoelectric point determined by isoelectric focusing: pH 4.21±0.2.
4) Optimal reaction pH: about pH 5.5 to 6.5.
5) pH stability: about pH 5 to 9 (37°C, 90 min. treatment).
6) Thermal stability: 55°C (10 minute treatment at pH 5).
7) Substrate specificity: the activity ratio to phosphatidylinositol is 10% or less of that of phosphatidyl choline.

The origin (roots) of PLB as a phospholipid processing agent of the present invention is not particularly limited as far as the PLB has the above characteristic features. When the PLB is a natural origin, the PLB is preferably microorganism origin, more preferably derived from the genus Candida, more specifically from Candida cylindracea. Further, the PLB as a phospholipid processing agent of the present invention may be an enzyme genetically modified based on an enzyme of natural origin as described above. The PLB produced by the genetic recombination technology is included in PLB as a phospholipid processing agent of the present invention, and it is desirable to have the characteristics as described above.

Further, the PLB includes an enzyme itself having an amino acid sequence represented by SEQ ID No. 1 or 2 or an amino acid sequence having 75% or more homology to the amino acid sequence represented by SEQ ID No. 1 or 2. Any enzyme with PLB activity is also included in the PLB as phospholipid processing agent of the present invention, and not particularly limited as far as it has the PLB activity. However, the homology to the amino acid sequence represented by SEQ ID No. 1 is preferably 80% or more, more preferably 85% or more, still more preferably 90% or more, particularly preferably 95% or more, most preferably 98% or more. Further, it is preferable that the phospholipid processing agent substantially does not decompose only phosphatidylinositol. The phrase "substantially does not decompose only phosphatidylinositol" is synonymous with one described above.

The term "homology" used in the present invention means the homology of amino acid sequences or DNA nucleotide sequences, which can be determined by a known method, for example, sequence comparison using a computer. Analysis software used is GENETEX WIN 5.2 (manufactured by Software Co., Ltd.). An exemplified computer program used in determination of homology may be, but not specifically limited to, a GCG program package containing GAP (Devereux, J. et., al., Nucleic Acids Research 12 (12): 387 (1984)), or a BLAST package (NCBI, or Altschul, S.F. et al., J. Mol. Biol., 215: 403-410 (1990)) and Smith-Waterman algorithm.

Further, the PLBs as the phospholipid processing agents of the present invention include any modified enzymes that are functionally equivalent and having an amino acid sequence represented by SEQ ID No. 1 or 2 with deletion, substitution, or addition of one to several of amino acids while having a PLB activity. The number of amino acids being deleted, substituted, or added is, but not specifically limited to, preferably one or more, more preferably two or more as the lower limit, and preferably 25 or less, more preferably 20 or less, still more preferably 15 or less, particularly preferably 10 or less, most preferably 5 or less as the upper limit. Further, preferably the phospholipid processing agent substantially does not decompose only phosphatidylinositol. The phrase "substantially does not decompose only phosphatidylinositol" is synonymous with one described above.

Amino acid sequences of five different lipases derived from Candida cylindracea are well known in the art. Any of the natural lipases may be used as a phospholipid processing agent of the present invention. Even an enzyme with deletion, substitution, or addition of one to several amino acids may be used in the following applications as far as the enzyme has a PLB activity.
Meanwhile, the phospholipid processing agent of the present invention is preferably further provided with a lipase activity. It is possible to act on triglyceride and hydrolyzes the phospholipid processing agent into free fatty acid at a molar ratio of three and glycerin at an equimolar ratio. If the phospholipid processing agent is applied to a mixture of phospholipid and triglyceride, they can be simultaneously hydrolyzed.

### 2. Method of manufacturing PLB

The PLBs for the phospholipid processing agents of the present invention can be easily commercially available in a form of enzyme products derived from the genus Candida and utilized. Alternatively, the enzymes may be produced from the products obtained by culturing the microorganisms of the genus Candida. Preferable examples of microorganisms of the genus Candida include, but not limited to, the strain Candida cylindracea. The PLB-producing strain may be mutant strains treated with ultraviolet rays and chemical mutagens. Further, genetic recombinants prepared by incorporating PLB genes described later in the form of being capable of high level expression into host cells may be used.
Culture medium components and culture conditions of culture of microorganisms are not particularly limited as far as strains are capable of PLB production. The culture conditions suitable for the enzyme production are generally those of cultures in nutritious culture media at medium incubation temperature under aerobic conditions. Good bacterial growth and enzyme production can be observed when the strain is cultured under any of such conditions. Specifically, for example, carbon sources for the culture medium include glucose, sucrose, fructose, lactose, maltose, corn starch, and potato starch. Examples of nitrogen sources include wheat bran, peptone, yeast extract, soybean meal, fat-free soybean meal, casein, cottonseed powder, fat-free cottonseed powder, gelatin, various amino acids (e.g., glutamic acid), ammonium sulfate, and urea etc. Those carbon sources and nitrogen sources may be appropriately combined and used. Of those, a preferred example includes a combination of soybean meal and glucose or a combination of fat-free soybean meal and glucose. Inorganic salts such as table salt, sodium phosphate, magnesium sulfate, and calcium chloride etc. are exemplified as other components to be used in the culture medium. Of those, salt, phosphate, or magnesium salt may be preferably used. Yeast of the genus Candida is inoculated in a sterile culture medium containing a carbon source, a nitrogen source, and inorganic salts and then incubated under aerobic conditions. The incubation temperature used is in the range of 22 to 33°C, preferably 26 to 30°C, particularly preferably 27 to 28°C. The incubation time is 30 to 60 hours depending on ventilation, agitation, or incubation temperature. PLB activity of the supernatant may be monitored and the incubation may be then terminated at the time of reaching the stationary phase.

The microorganism used in the present invention can secrete and produce an enzyme, PLB, for a phospholipid processing agent of the present invention under the above culture conditions. The supernatant of the culture medium as a material for the production of the enzyme can be obtained by separating from fungus body. The PLB contained in the culture supernatant obtained by centrifugal separation and filtration procedures can be refined by salt precipitation with ammonium sulfate and precipitation with an organic solvent such as acetone or ethanol, but preferable purification is with acetone precipitation. An enzyme may be concentrated by ultrafiltration prior to those precipitation procedures. Further, if required, a purified enzyme can be obtained by purification and/or concentration of the enzyme to a desired level by well-known procedures such as ion exchange chromatography, gel filtration, hydrophobic chromatography, and affinity chromatography. The higher the degree of purification of an enzyme contained in the phospholipid processing agent used in the present invention becomes, the more the phospholipid can be efficiently decomposed. However, the enzyme even in crude state can be used depending on the purpose. Even if the enzyme is a mixture of enzyme fractions A and B fractionated by ion exchange chromatography, it can be used.

When the PLB as the phospholipid processing agent of the present invention is a modified enzyme modified enzymes that are functionally equivalent, a person skilled in the art can obtain such an enzyme on the basis of information about the base sequence of a gene of SEQ ID No. 3 that encodes an enzyme having an amino acid sequence represented by SEQ ID No. 1 in the sequence listing. Note that, gene recombinant technologies may be carried out according to the well-known method (for example, manuals for genetic recombination technologies, such as Sambrook, J. et al., "molecular Cloning - A Laboratory Manual", Cold Spring Harbor Laboratory, NY, 1989).

For example, the modified enzyme can be obtained as follows: a suitable primer or a suitable probe is designed from information on gene sequence of SEQ ID No. 3 in the sequence listing. The primer or the probe is used together with a sample derived from a living organism of interest to carry out a polymerase chain reaction (PCR) method or a hybridization method, thereby obtaining a gene of interest. Subsequently, a gene modification is carried out by a method commonly used in gene alteration, such as a method of inducing a site-specific mutation (Mark, D. F., Proc. Natl. Acad. Sci. USA, 81, 5662-5666, 1984). Then, the modified gene is expressed in a suitable expression system such as one using Saccharomyces or Pichia as a host to obtain the modified enzyme. A method described in Example 2 can be used for confirming whether the modified enzyme has a PLB activity. It is preferable that the phospholipid processing agent containing the modified enzyme substantially does not decompose only phosphatidylinositol in a phospholipid mixture. Further, it is preferable to have a lipase activity.

### 3. Method of manufacturing phosphatidylinositol (PI) and/or glyceryl phosphoryl choline (GPC)

The present invention provides a method of simultaneously and efficiently manufacturing high-purity PI and GPC. In addition, a method of individually and efficiently manufacturing high-purity PI or GPC can also be provided. The phospholipid processing agent of the present invention has phospholipid specificity for selectively hydrolyzing phospholipids except PI in a phospholipid mixture. So, high-purity PI can be selectively manufactured. Further, GPC of high-purity can be manufactured simultaneously with the manufacture of PI by decomposing the soybean phospholipid containing PC as a main component using the phospholipid processing agent of the present invention. The acquisition of PI and GPC of high-purity is extremely useful as functional phospholipid or functional phospholipid raw materials.
PI can also be obtained by acting PLC on phospholipid (see Patent Document 2 as described above) but it results in no generation of GPC. The GPC generated as a reaction product by the present invention is a material expected to be useful for dementia prevention and the like, so the present invention, which uses the action of PLB, can be advantageous in this point as well.

Hereinafter, the methods of manufacturing the respective components will be described in detail.

### (a) Method of manufacturing phosphatidylinositol (PI)

PLB as a phospholipid processing agent of the present invention is extremely useful in application of processing naturally-occurred phospholipids. The present invention provides a method of efficiently manufacturing PI of high-purity. Phospholipid is a structural component of a cell membrane and responsible for an important physiological function. In particular, PI is intimately involved in intra- and extracellular signal transduction in the cell membrane. The source of the supply of phospholipids in medicine / food applications is mainly soybeans, chicken egg yolk, fish eggs, or the like. Those phospholipids are usually obtained as mixtures of a plurality of molecular species, such as phosphatidyl choline (PC), phosphatidyl ethanolamine (PE), phosphatidylserine (PS), phosphatidic acid (PA), and phosphatidylinositol (PI). In order to separate PI from those phospholipid mixtures, organic solvent fractionation, chromatographic separation with a carrier such as a silica gel or alumina, or the like have been commonly used. Those methods can efficiently obtain comparatively sufficient contents of PC and PE but extremely insufficient to obtain phospholipid components such as small content of PI. Meanwhile, there is a need of using a large amount of a halogen-based organic solvent such as chloroform. Thus, applications of the resulting products are limited. In order to effectively produce PI from phospholipids containing PC, PE, PS, PA, PI, phospholipids such as PC, PE, PA, and PS except PI may be selectively hydrolyzed to allow PI to remain.

PI can be efficiently obtained by selectively leaving only PI from a phospholipid mixture by allowing the phospholipid processing agent of the present invention to act on the phospholipid mixture. The phospholipid mixture may be dispersed in water using a homogenizer or the like in advance or may be forcibly agitated to prepare a homogeneous aqueous solution. The concentration of the phospholipid may be any concentration as far as PLB can act on the phospholipid mixture dispersed in water and is in the range of 1 to 20%, preferably 5 to 10%, particularly preferably 6 to 8%. The pH of the reaction may be any pH as far as PLB can act on the phospholipid mixture and is preferably in the range of pH 3 to 10, or about pH 5.5 to 6.5 at which the PLB activity reaches the maximum. The pH is particularly preferably about pH 6.

It is preferable to use a buffer to keep the reaction at a constant pH. The type of a buffer is not specifically limited as far as it has a buffer capacity at the range of pH 5.5 to 6.5. In view of food application, an acetate buffer is preferably used. In addition, the pH of the reaction solution can be adjusted within a preferred range by suitable addition of a NaOH solution in the reaction.
The concentration of PLB used as a phospholipid processing agent of the present invention may be in the range of 1,000 to 100,000,000 units per kg of phospholipid, preferably is in the range of 2,000 to 5,000,000 units per kg of phospholipid.
The PLB to be used may be added in the form of an aqueous solution, or may be in the form of being fixed on an insoluble carrier such as Celite or ion exchange resin.
The enzyme reaction may be carried out at any temperature as far as PLB is not inactivated. The upper limit of the temperature is preferably 60°C or less, more preferably 45°C or less, and the lower limit of the temperature is preferably 10 °C or more, more preferably 30°C or more.
The reaction time varies depending on the enzyme reaction conditions and is generally 1 to 150 hours. The reaction may be terminated depending on a qualitative understanding of the remaining amount of a substrate.
After the end of the reaction, the deactivation of PLB may be carried out with thermal treatment, pH treatment, or the like without any trouble.
The silica gel thin layer chromatography (TLC) method is the simplest and easiest to confirm the state of the hydrolysis reaction. When the TLC is used and iodic color development is then used to qualitatively confirm the remaining amount of the substrate, the reaction may be terminated at the time when almost no spot indicating any phospholipid except PI on the TLC can be seen.
In products resulting from the reaction, free fatty acid, glyceryl phosphoryl choline (GPC), glyceryl phosphoryl ethanolamine (GPE), glycerophosphoric acid (GP), and the remaining unreacted PI are present in mixture. The PI can be recovered from the mixture using an organic solvent such as chloroform, ethanol, methanol, and hexane. In view of food application, particularly, ethanol, hexane, or a mixture solvent thereof is preferably used. The pH of the reaction may be changed before the extraction for the purpose of improving liquid separation in the extraction process.
The free fatty acids and PI are soluble in an organic solvent. GPC, GPE, and the like are insoluble in an organic solvent such as hexane but soluble in water. The solvent is to be removed from the PI and free fatty acids that are recovered in the organic solvent layer by procedures such as vacuum concentration. PI is insoluble in a water-soluble or water-miscible alkylcarbonyl solvent. The free fatty acid is soluble in any of those solvents. Thus, PI can be recovered as precipitation. The water-soluble or water Miscible alkylcarbonyl solvent may be acetone or methyl ethyl ketone etc. A preferable example is acetone. PI may be recovered by the centrifugal separation or the solid-liquid separating procedures of a filtration method.

The phospholipid mixture is not particularly limited. Phospholipids derived from chicken egg yolk, phospholipids derived from roe such as salmon roe, or phospholipids derived from soybean can be used. The phospholipid derived from chicken egg yolk or the phospholipid derived from soybean is preferable from an aspect of the stable supply of source phospholipid, and the phospholipid of the soybean origin is particularly preferable. The phospholipid processing agent of the present invention may have a strong lipase activity in addition to the PLB activity. However, having the lipase activity is not a problem at all to produce PI and glyceryl phosphoryl choline from the soybean phospholipid. The soybean phospholipid is also supplied as a mixture with triglyceride. When such a mixture is used as a raw material, both the triglyceride and the phospholipid can be simultaneously hydrolyzed. In this regard, it is significant to have both the lipase activity and the PLB activity. When the PLB activity is high but the lipase activity is small or absent, additional lipase may be added to simultaneously decompose the triglyceride and phospholipid.

According to the above method of the present invention, high-purity PI is provided. The purity of PI is not particularly limited as far as PI has high purity. However, the purity of PI in the total phospholipid is preferably 50 mol% or more, more preferably 60mol% or more, further preferably 70 mol% or more, particularly preferably 80 mol% or more, and most preferably 85 mol% or more as the lower limit. High-purity PI is useful as a functional phospholipid. As PI involves inositol that is rich in hydroxyl groups in the molecule, it shows good dispersability or solubility in water compared to other phospholipids such as PC and PE, PI and has, for example, extremely different solubility of other lipid components, and hence, it may be advantageous in that it can be applied to a wide range of applications as phospholipid.

### b) Manufacture of glyceryl phosphoryl choline (GPC)

PLB as a phospholipid processing agent of the present invention is extremely useful in application of processing of naturally-occurred phospholipid. Thus, the present invention offers a method of efficiently manufacturing high-purity GPC.
The method of allowing the phospholipid processing agent of the present invention to act on a phospholipid mixture is the same as that of the method of manufacturing the above PI. The reaction time may be generally in the range of 5 to 20 hours. The reaction may be terminated at the time when the concentration of GPC in the reactant reaches the maximum, although it depends on the concentration of enzyme to be used.
The same organic solvent as one mentioned above, such as chloroform, ethanol, methanol, or hexane, is added to a reaction solution of PLB obtained by allowing the phospholipid processing agent of the present invention act on the phospholipid mixture. An aqueous layer remained after extraction of lipid components (including free fatty acids) contains GPC, GPE, GP, or free enzymes. The aqueous solution containing GPC, GPE, or GP is directly subjected to vacuum concentration, thereby obtaining a syrupy solution. In advance to the concentration, it is preferable to treat the aqueous solution containing GPC, GPE, or GP with activated carbon in terms of removing coloring components and the phospholipid processing agent of the present invention. Higher-purity GPC may be obtained using a cation ion exchange resin or the like.

The phospholipid composition used in the method of manufacturing GPC of the present invention is not particularly limited as far as it has a high PC content. Phospholipids derived from chicken egg yolk, phospholipids derived from roe such as salmon roe, or phospholipids derived from soybean can be used. Preferably, the phospholipids derived from chicken egg yolk and the phospholipids derived from soybean are preferable from an aspect of the stable supply of source phospholipid. In particular, the phospholipids derived from soybean are preferable. The above method of the present invention offers high-purity GPC. The purity of GPC is not particularly limited as far as it is high purity. The purity of GPC is preferably 45 wt% or more, more preferably 50 wt% or more as the lower limit.

### 4. Method of manufacturing lysophosphatidylinositol

Next, a method of manufacturing lysophosphatidylinositol will be described.
The method of manufacturing high-purity lysophosphatidylinositol of the present invention includes the following steps:
Step (1): step of subjecting high-purity PI to a hydrolysis reaction with an enzyme having phospholipase A1 or phospholipase A2; and
Step (2): step of removing free fatty acid by washing with a mixture solvent containing one or two or more kinds of solvents in which free fatty acid is dissolved while lysophosphatidylinositol cannot be dissolved and separating lysophosphatidylinositol from free fatty acid, which is a reaction product, to recover the lysophosphatidylinositol.
Hereinafter, the details will be described.

Enzymes having phospholipase A1 or phospholipase A2 activity used in the manufacture of lysophosphatidylinositol of the present invention are not limited as far as the enzymes are those acting on PI to convert the PI into lysophosphatidylinositol. Examples of the enzymes include: phospholipase A2 (Lectitase manufactured by Novozym Co., Ltd. or Lipomod 699L manufactured by Genencor Kyowa Co., Ltd.) included in an enzyme mixture, pancreatin, derived from the pig pancreas; phospholipase A2 (Lizomax PF manufactured by Genencor Kyowa Co., Ltd.) derived from Streptomyces violaceoruber; phospholipase A1 (Phospholipase A1 manufactured by Sankyo LifeTech Co., Ltd.) etc. derived from Aspergillus oryzae. Of those, preferable examples may be phospholipase A2 (Lectitase manufactured by Novozym Co., Ltd.) contained in the enzyme mixture, pancreatin, derived from the pig pancreas and phospholipase A1 (Phospholipase A1 manufactured by Sankyo LifeTech Co., Ltd.) derived from Aspergillus oryzae.

The enzyme reaction in the manufacture of lysophosphatidylinositol of the present invention may be carried out in a way where an enzyme and a substrate be brought into contact with each other in aqueous medium or under wet conditions. The pH of the reaction is not limited as far as it is within the range where the enzyme reaction is carried out. For example, a preferable range of pH is pH 6.5 to 9.0.
The reaction temperature is not limited as far as it is within the range where the enzyme reaction is carried out. For example, the temperature is preferably in the range of 10 to 70°C, more preferably in the range of 30 to 60°C.
The reaction may be terminated when the reaction becomes a desired state where the reaction of a compound suitable to follow the reaction, such as a decrease in PI or an increase in lysophosphatidylinositol, is followed using, for example, the analysis of high performance liquid chromatography, thin-layer chromatography. The desired state may be, for example, a reaction-terminated state, a steady state, or a state of producing a sufficient amount of lysophosphatidylinositol, which may be determined from various viewpoints of industrial production.
Examples of the reaction time may be one hour to ten days.
The amount of enzyme used in the reaction may be an amount enough to sufficiently progress the reaction, but there is no advantage in using more than required amount from the viewpoint of industrial production. For example, when 1,000 U/g of enzyme is used, a preferable example may be 0.05 to 50 wt% with respect to the substrate. In addition, calcium ion (calcium chloride) or the like may be added as an enzyme stabilizer. Further, the enzyme may be deactivated by the conventional method after terminating the enzyme reaction.
The activation means may be a heat treatment (at 50 to 90°C for 10 minutes to 2 hours), a pH treatment (at pH 1 to 4 or pH 8 to 12 for 10 minutes to 2 hours), or the like. Further, after terminating the reaction or after deactivating the enzyme, the generated free fatty acid may be removed by washing with a solvent such as acetone in which fatty acid can be dissolved but lysophosphatidylinositol cannot be dissolved.

### 5. Method of manufacturing glyceryl phosphoryl inositol

Next, a method of manufacturing glyceryl phosphoryl inositol will be described.
The method of manufacturing high-purity glyceryl phosphoryl inositol of the present invention includes the following steps:
Step (1): step of subjecting high-purity PI to a hydrolysis reaction with an enzyme having a phospholipase B activity; and
Step (2): step of separating glyceryl phosphoryl inositol from free fatty acid (reaction product) to recover the glyceryl phosphoryl inositol.
Hereinafter, the method will be described in detail.

The enzyme having phospholipase B activity used in the production of glyceryl phosphoryl inositol of the present invention is not limited as far as it acts well on PI and converts the PI into glyceryl phosphoryl inositol. Examples of such an enzyme include enzymes derived from the genus Penicillium, yeast, and the like.
The enzyme reaction of the present invention may be carried out in a way that an enzyme and a substrate may be brought into contact with each other in aqueous medium or under wet conditions. The pH of the reaction is not limited as far as it is within the range where the enzyme reaction is carried out. For example, a preferable range of pH is pH 3.5 to 8.0. When phospholipase B derived from Penicillium notatum is used as an enzyme, about pH 4.0 to 5.0 is particularly preferable.
The reaction temperature is not limited as far as it is within the range where the enzyme reaction is carried out. For example, the temperature is preferably in the range of 10 to 70°C, more preferably in the range of 30 to 60°C.
The reaction may be terminated when the reaction becomes a desired state where the reaction of a compound suitable to follow the reaction, such as a decrease in PI or an increase in glyceryl phosphoryl inositol, is followed using, for example, the analysis of high performance liquid chromatography, thin-layer chromatography. The desired state may be, for example, a reaction-terminated state, a steady state, or a state of producing a sufficient amount of glyceryl phosphoryl inositol, which may be determined from various viewpoints of industrial production. Examples of the reaction time may be one hour to ten days.
The amount of enzyme used in the reaction may be an amount enough to sufficiently progress the reaction but there is no advantage in using more than required amount from the viewpoint of industrial production. For example, when 3,800 U/g of enzyme is used, a preferable example may be 0.05 to 5 wt% with respect to the substrate. In addition, calcium ion (calcium chloride etc.) may be added as an enzyme stabilizer. Further, the enzyme may be deactivated by the conventional method after terminating the enzyme reaction. The activation means may be a heat treatment (at 50 to 90°C for 10 minutes to 2 hours), a pH treatment (at pH 1 to 4 or pH 8 to 12 for 10 minutes to 2 hours), or the like.
Further, after terminating the reaction or deactivating the enzyme, the generated free fatty acid may be subjected to extraction removal with a solvent such as hexane, and an aqueous layer may be then subjected to vacuum concentration, followed by storing in an aqueous solution containing a high concentration of glyceryl phosphoryl inositol or storing as freeze-dried powder. Alternatively, glyceryl phosphoryl inositol in the aqueous layer may be directly adsorbed in an ion exchange resin or the like, followed by washing, After that, the free fatty acid may be further purified by using high-concentrated salt solution of various pH. Further, if required, any coloring component may be adsorbed on activated carbon to carry out decoloration.

### 6. Use of high-purity PI and high-purity GPC

The PI obtained by the method of manufacturing PI of the present invention uses PLB derived from Penicillium notatum or Dictyostelium discoidoum, which can well hydrolyze PI, and then may be used as a raw material in the production of high-purity glyceryl phosphoryl inositol. In addition, GPC obtained by the method of manufacturing the GPC of the present invention can be used as a raw material at the time of producing high-purity choline phospholipid (i.e., functional phospholipid) containing a fatty acid by chemical or enzymatic ester synthesis of GPC with free fatty acid or fatty acid ester.

### 7. Food, medicine, or cosmetic containing phosphatidylinositol (PI), glyceryl phosphoryl choline (GPC), lysophosphatidylinositol, or glyceryl phosphoryl inositol

The contents of PI, GPC, lysophosphatidylinositol, and glyceryl phosphoryl inositol in foods and medicines are not limited. The content of each of those may preferably correspond to an intake of PI, GPC, lysophosphatidylinositol, or glyceryl phosphoryl inositol of 1 to 10,000 mg, preferably 10 to 5,000 mg, and more preferably 30 to 1,000 mg per day.
Foods containing PI, GPC, lysophosphatidylinositol, or glyceryl phosphoryl inositol of the present invention are not limited as far as they include any of those components. Examples of the foods include supplements (e.g., powder, granule, soft capsule, hard capsule, tablet, chewable tablet, quick-disintegrating agent, syrup, solution etc.), drinks (e.g., tea, carbonated beverage, lactic drink, and sport drink etc.), confectionery (e.g., gummy, jelly, gum, chocolate, cookie, and candy etc.), oils, oil/fat food (e.g., mayonnaise, dressing, butter, cream, and margarine etc.), ketchup, source, fluid food, dairy products (e.g., milk, yogurt, and cheese etc.), bread, noodles (e.g., udon, soba noodles, ramen, pasta, fried noodles, kisimen, soumen, hiyamugi, and rice vermicelli etc.), soups (e.g., miso soup, corn soup, and consomme etc.), and dried seasonings for sprinkling over rice etc.

The foods containing PI, GPC, lysophosphatidylinositol, or glyceryl phosphoryl inositol of the present invention may be added with, if required, any of various nutrients, various vitamin groups (e.g., vitamin A, vitamin B1, vitamin B2, vitamin B6, vitamin B12, vitamin C, vitamin D, vitamin E, and vitamin K), various minerals (e.g., magnesium, zinc, iron, sodium, potassium, selenium, titanium oxide), dietary fibers, various saccharides (e.g., cellulose, dextrin, and chitin), various polyunsaturated fatty acids (e.g., arachidonic acid, docosahexaenoic acid, eicosapentaenoic acid, and docosapentaenoic acid), various conjugate fatty acids (e.g., conjugated linoleic acid, conjugated linolenic acid, conjugated arachidonic acid, conjugated DHA, conjugated EPA, and conjugated DPA), various phospholipids (e.g., lecithin, PA, PS, PE, phosphatidyl glycerol, PC, and phosphatidyl DHA), various glycolipids (e.g., cerebroside), various carotenoids (e.g., β-carotene, lycopene, astaxanthin, β-cryptoxanthin, capsanthin, lutein, and zeaxanthin), various flavonoids (e.g., quercetin, luteolin, and isoflavone), various amino acids (e.g., glycine, serine, alanine, glutamine, valine, leucine, isoleucine, lysine, arginine, aspartic acid, glutamic acid, tryptophan, phenylalanine, histidine, proline, methionine, and cysteine), other various nutrients (e.g., oxidized form coenzyme Q10, reduced form coenzyme Q10, carnitine, sesamin, α-lipoic acid, inositol, D-chiro inositol, pinitol, taurine, glucosamine, chondroitin sulfate, S-adenosylmethionine, curcumin, γ-orizanol, glutathione, γ-aminobutyric acid, synephrine, pyrroloquinoline quinone, catechin, and capsaicin), various dispersing agents, stabilizing agents such as various emulsifiers, various sweeteners (e.g., sorbitol and sucrose), various taste ingredients (e.g., citric acid and malic acid), flavors, royal jelly, honey, beeswax, propolis, agaricus, ginseng, bioperine, and the like. Further, any of herbs, such as peppermint, bergamot, chamomile, and lavender, may be added. Further, any of materials, such as teanin, dehydroepiandrosterone, and melatonin, may be combined.

The medicines containing PI, GPC, lysophosphatidylinositol, or glyceryl phosphoryl inositol of the present invention are not limited as far as medicines contain any of these components. The formulations of the medicine include powder, granule, pill, soft capsule, hard capsule, tablet, chewable tablet, quick-disintegrating agent, syrup, solution, suspending agent, suppository, ointment, cream, gel, mucilage, inhalant, and injectable etc. Those formulations are prepared according to the conventional methods. However, because of the insolubility of PI in water, it may be dissolved in any of non-hydrophilic organic solvents, such as vegetative oils and animal oils. Alternatively, PI may be dispersed or emulsified in an aqueous solution using a homogenizer (high-pressure homogenizer) together with an emulsifier, a dispersing agent, or a surfactant. Further, for improving the absorbency of PI, PI may be used after being pulverized to about 1 micron particle diameter on average in the presence or absence of excipients (e.g., Arabian gum, dextrin, or casein).

The additives which can be used for formulation include, for example: soybean oil, safflower oil, olive oil, germ oil, sunflower oil, beef tallow, animal oils such as sardine oil; polyalcohol such as polyethylene glycol, propylene glycol, glycerin, and sorbitol; surfactants such as sorbitan fatty acid ester, sucrose fatty acid ester, fatty acid ester of glycerin, and polyglyceryl fatty acid ester; diluting agents such as purified water, lactose, starch, microcrystalline cellulose, D-Mannitol, maltose, lecithin, Arabian gum, dextrin, a sorbitol solution, and a carbohydrate solution; sweeteners; colorants; pH adjustors; and flavors. In addition, the liquid preparation may be in the form of being dissolved or suspended in water or any of other appropriate media when being dosed. Further, the tablets or the granules may be coated with well-known methods.

When it is administered in the form of injectable, such as intravenous, intraperitoneal, intramuscular, subdermal, transdermal, intraarticular, intrasynovial, intraalveolus, intraperiosteal, sublingual, or intraoral administration is preferable. Among them, in particular, intravenous administration or intraperitoneal administration is preferable. An intravenous administration may be any one of drip infusion and porous administration.
Cosmetic containing PI, GPC, lysophosphatidylinositol or glyceryl phosphoryl inositol of the present invention include creams, milky lotion, lotions, micro emulsion essence, poultice, and bath powder etc. and fragrance or the like may be mixed.
The present invention will now be described with reference to examples, but the invention is not limited by the following examples.

### Examples

### [Example 1] Method of manufacturing PLB

To a 30-liter volume jar fermenter with 20 liters of a sterilized medium containing 3% fat-free cottonseed powder, 0.3% table salt, 0.2% dipotassium phosphate, 0.2% potassium phosphate, 0.1% magnesium sulfate, and antifoam 0.3%, 100 ml of a culture medium of Candida cylindracea strain ATCC14830 preincubated in the same medium (28°C for 4 days) was sterilely inoculated. The incubation was carried out at 28°C with aeration (20 litters per minute) of sterilized air while stirring at 300 rpm. It was confirmed that PLB had the maximum activity (0.2 U/ml) after 50 hours.
The resulting culture medium was centrifuged at 5,000 rpm for 10 minutes, resulting in 16 liters of the supernatant. The supernatant was concentrated by ultrafiltration. A sediment generated by the addition of 9 liters of cold acetone to 3 liters of the resulting concentrate was centrifuged at 5,000 rpm for 10 minutes to collect the precipitation, followed by dissolving the precipitation in 2 litters of a 10 mM Tris hydrochloric acid buffer solution (hereinafter, abbreviated as Tris-HCl) (pH 7.5) with 2 M sodium chloride. An insoluble substance was removed by centrifugation and PLB was then adsorbed by passing through a column previously filled with octyl sepharose (bed volume; 300 ml). Subsequently, the column was washed with 10 mM Tris-HCl (pH 7.5) containing 2 M sodium chloride and 10 mM Tris-HCl (pH 7.5). PLB adsorbed on the column was eluted with 10 mM Tris-HCl (pH 7.5) containing 2% Adecatol SO120 (purification with hydrophobic chromatography). Then, the eluate (1 liter) was passed through DEAE-sepharose column (bed volume; 200 ml) previously equilibrated with 10 mM Tris-HCl (pH 7.5) to adsorb PLB. The column was washed with the above buffer and an enzyme was then eluted by a concentration gradient of 0 to 0.3 M sodium chloride. PLB enzyme fraction A was obtained at about 0.1 M of sodium chloride and PLB enzyme fraction B was obtained at about 0.25 M of sodium chloride (separation purification with ion-exchange chromatography).
N-terminal amino acid sequences of enzyme fraction A and enzyme fraction B were defined by an amino acid sequencer. As a result, the amino acid sequence of enzyme fraction A was a sequence of SEQ ID No. 1 and the amino acid sequence of enzyme fraction B was a sequence of SEQ ID No. 2. Further, a homology analysis was carried out using GENETEX WIN 5.2 (manufactured by Software Co., Ltd.) to analyze the homology of enzyme fraction B with respect to enzyme fraction A, resulting in 88.5%.

### [Example 2] Method of measuring PLB activity

The enzyme activity of PLB can be checked by carrying out an enzyme method to measure GPC generated from phosphatidyl choline. Namely, 0.5 ml of a reaction solution consisting of 0.05 ml of 10 mM egg yolk phosphatidyl choline, 0.025 ml of 1M-calcium chloride, 0.05 ml of 0.2% TODB, 0.05 ml of 0.2% 4-aminoantipyrine, 0.1 ml of 50 U/ml monoglyceride lipase, 0.1 ml of 300 U/ml glycerophosphoric acid oxidase, 0.025 ml of 6 U/ml GPC phosphadiesterase, and 0.05 ml of 100 U/ml peroxidase dissolved in 0.5 ml of an 1 M MES-NaOH buffer (hereinafter, abbreviated as MES-NaOH) (pH 6) with 3% Triton X-100 was pre-heated at 37°C for 2 to 3 minutes. Subsequently, the reaction solution was diluted with 10 mM Tris-HCl (pH 7.5) containing 0.05% BSA, and then the reaction solution was added with 25 µl of a PLB solution (0.03 to 0.15 U/ml), which was diluted with the same dilution buffer to initiate the reaction. After exactly 10 minutes, the reaction was terminated with the addition of 1 ml of 0.5% SDS, and then, the absorbance thereof at 550 nm was measured.
Note that, the activity for releasing 1 micromole of GPC per minute was defined as one unit.

### [Example 3] Method of measuring lipase activity

The lipase activity measurement was carried out by converting monoglyceride generated using diglyceride as a substrate into glycerin with monoglyceride lipase, followed by measuring with an enzyme method. Namely, 0.5 ml of a reaction solution consisting of 0.05 ml of 10 mM 1,2 diglyceride, 0.025 ml of 0.5 M calcium chloride, 0.025 ml of 0.05 M magnesium chloride, 0.05 ml of 0.05 M ATP, 0.05 ml of 10 U/ml monoglyceride lipase, 0.05 ml of 5 U/ml glycerol kinase, 0.25 ml of 400 U/ml glycerophosphoric acid oxidase, 0.025 ml of 100 U/ml of peroxidase, 0.025 ml of 0.3% TOOS, and 0.025 ml of 0.3% 4-aminoantipyrine, which were dissolved in 0.1 mM of 1 M MES-NaOH (pH 6.0) with 3% Triton X-100, was added with 25 µl of an enzyme solution (0.03 to 0.15 U/ml) diluted with 10 mM Tris-HCl containing 0.05% BSA, followed by carrying out the reaction at 37°C for 10 minutes. Subsequently, the reaction was terminated with 0.5% SDS and the absorbance thereof at 550 nm was then measured. Note that, the activity for releasing 1 micromole of glycerin per minute was defined as one unit of lipase activity.

### [Example 4] Purification by gel filtration chromatography of PLB fraction A

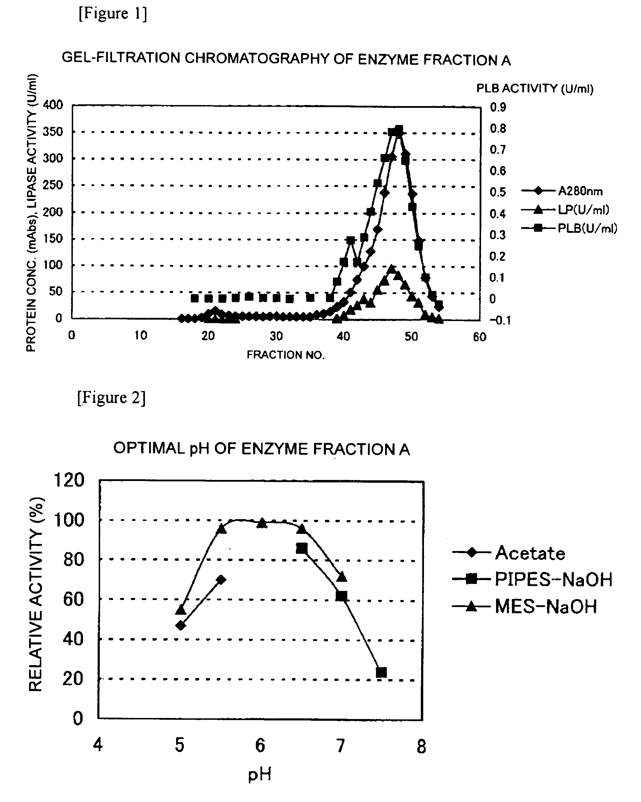

PLB-active enzyme fraction A obtained in Example 1 was concentrated by a centrifugal ultrafiltration device. Subsequently, the concentrated solution was subjected to gel-filtration chromatography (SuperDex 75) previously equilibrated with 10 mM Tris-HCl (pH 7.5) containing 0.5 M sodium chloride and then separation at a flow rate of 0.5 ml per minute, thereby obtaining an active fraction.
In each fraction, the PLB activity and the lipase activity were obtained by the methods described in Examples 2 and 3. A protein concentration was measured using an absorbance of 280 nm. The results of the PLB activity, the lipase activity, and the protein concentration of each fraction are shown in Fig. 1.
Consequently, an elution pattern in which the protein concentration measured at 280 nm, the lipase activity and the PLB activity agreed with each other. It was found that the protein having the lipase activity and the PLB activity was an identical enzyme protein.

### [Example 5] Properties of PLB

### 5-1 (Optimum pH of reaction)

In the composition of the reaction solution shown in Example 2, the enzyme activities of enzyme fraction A at various pH using acetate buffer (hereinafter, abbreviated as Acetate), PIPES-NaOH buffer (hereinafter, abbreviated as PIPES-NaOH), or MES-NaOH were measured. The relative activities of enzyme fraction A in each buffer with respect to the activity obtained using MES-NaOH pH 6.0 were obtained. The results are shown in Fig. 2.
As shown in Fig. 2, MES-NaOH showed the maximum enzyme activity at about pH 6. In addition, PIPES-NaOH and Acetate are also expected to show the maximum enzyme activity at about pH 6.

### 5-2 (pH stability)

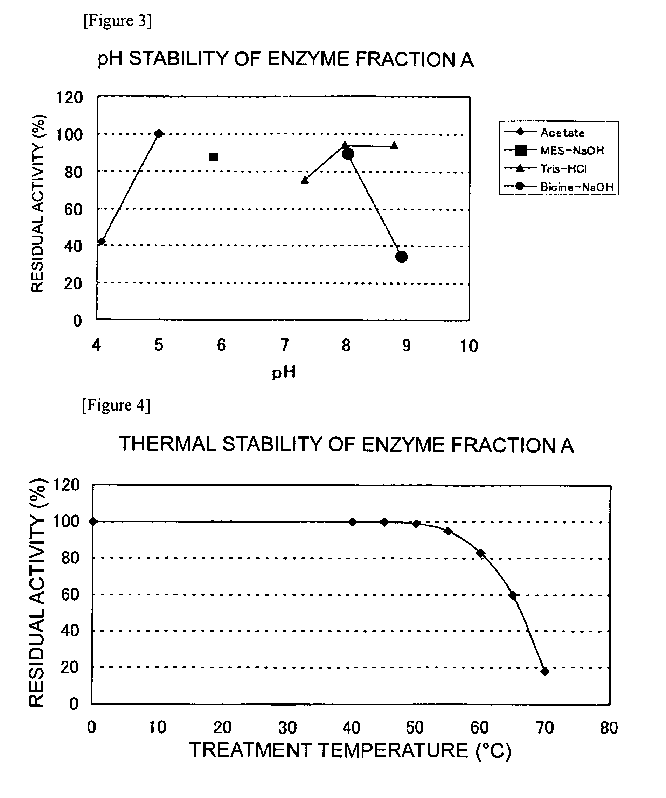

Enzyme fraction A was dissolved so as to be 5 U/ml in 10 mM of each buffer of Acetate, MES-NaOH, Tris-HCl, or Bicine-NaOH buffer (hereinafter, abbreviated as Bicine-NaOH) and then left standing at 37°C for 90 minutes. Subsequently, the PLB activity was measured on the basis of the method of Example 2, followed by measuring relative residual activity at each pH with respect to the activity in Acetate pH 5. The results are shown in Fig. 3.
As shown in Fig. 3, the pH stability was found in a wide range of pH 5 to pH 8.

### 5-3 (Thermal stability)

Enzyme fraction A was dissolved so as to be 5 U/ml in 10 mM of MES-NaOH (pH 6.0) and then subjected to the PLB activity measurement after 10 minute heating at a temperature range of 0 to 70°C by the method shown in Example 2. The results are shown in Fig. 4. The relative residual activity was 90% or more with the treatment at a temperature of up to 55°C when the PLB activity of enzyme fraction A preserved in frozen storage was defined as 100%. Consequently, it was found to be a heat-stable enzyme.

### 5-4 (Substrate specificity)

The activity of each of PLBs (enzyme fraction A and enzyme fraction B), which were two different phospholipid processing agents obtained by ion-exchange chromatography of Example 1, was measured by the method of measuring the PLB activity as described in Example 2 with respect to the respective phospholipids compared to PC. In other words, instead of the reaction composition of lecithin shown in Example 2, PE, PI, or PA was used in the measurement of activity to determine the relative activity to PC. The results are shown in Table 1. As a result, both enzyme fraction A and enzyme fraction B showed smaller relative activities to PC, compared with those of other phospholipids. The phospholipid processing agent of the present invention was found to have substrate specificity such that the activity to PI is smaller than the activity to any of other phospholipids. The result in Table 1 shows that each fraction has PLB activity, so the phospholipid processing agent may be a combination of both fractions.

**[Table 1]**

| Substrate | Relative activity (%) | |
|---|---|---|
| | Enzyme fraction A | Enzyme fraction B |
| Phosphatidyl choline (PC) | 100 | 100 |
| Phosphatidyl ethanolamine (PE) | 32.1 | 45.8 |
| Phosphatidic acid (PA) | 20.1 | 30.5 |
| Phosphatidylinositol (PI) | 5.4 | 4.2 |

### 5-5 (Molecular weight)

The purified enzyme fraction A obtained by the method described in Example 1 was subjected to a SDS-polyacrylamide electrophoresis and resulted in a single band. The molecular weight of the product was 53 kilodaltons with reference to the known molecular weight of protein as a marker.

### 5-6 (Isoelectric point)

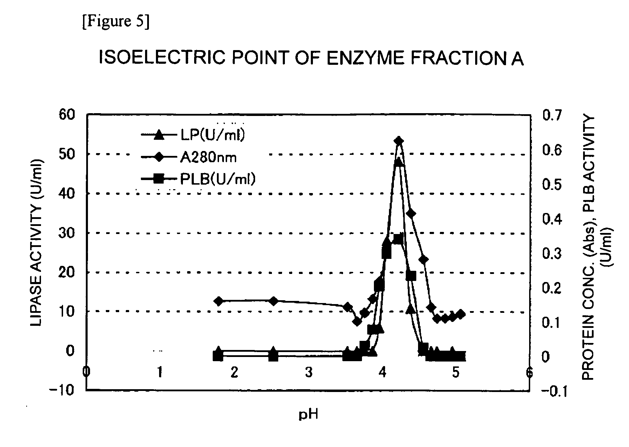

The purified enzyme fraction A obtained by the method described in Example 1 was subjected to a measurement of an isoelectric point of enzyme fraction A with an isoelectric focusing carried out by forming a pH gradient using Carrier Ampholytes. Further, the purified enzyme fraction A was subjected to a measurement of a protein concentration with absorbance at 280 nm. The relationship between the isoelectric point and the enzyme concentration of enzyme fraction A is illustrated in Fig. 5. As a result, the pH of the protein measured with 280 nm absorbance was completely coincident with lipase activity and PLB activity, and the pH value (isoelectric point) was 4.21. As is apparent from Fig. 5, the protein showed the same peak as that of PLB and lipase, so PLB and lipase were found to be the same enzyme protein.

### [Example 6] Quantitative test for PI

0.5 ml of coloring solution containing 0.1 ml of 1 M Tris-HCl (pH 8), 0.05 ml of 10 mM NAD, 0.05 ml of 10 mM magnesium chloride, 0.05 ml of 2% Triton X-100, 0.05 ml of 50 U/ml PI-specific phospholipase C, 0.05 ml of 50 U/ml alkaline phosphatase,0.05 ml of 50 U/ml inositol dehydrogenase, 0.05 ml of 5% nitroblue tetrazolium, and 0.15 ml of purified water was added with 0.02 ml of a PI solution prepared by dissolving a test sample (1 to 3 mg/ml) containing PI in 2% Triton X-100, thereby carrying out a reaction at 37°C for 10 minutes. The reaction was terminated by 0.5 ml of 0.5% SDS and the absorbance was then measured at 550 nm. An aqueous solution of a known concentration of inositol was used for calibration and then the amount of PI was quantitatively assayed.

### [Example 7] Quantitative assay of GPC

0.5 ml of reaction solution containing 0.1 ml of 1 M Tris-HCl (pH 7.5), 0.025 ml of 1M calcium chloride, 0.05 ml of 0.2% TODB, 0.05 ml of 0.2% 4-aminoantipyrine, 0.025 ml of 6 U/ml glyceryl phosphoryl choline phosphodiesterase, 0.05 ml of 100 U/ml peroxidase, 0.05 ml of 200 U/ml choline oxidase, and 0.15 ml of purified water was added with 25 µl of a sample containing GPC (0.3 to 0.9 mg/ml). After reacting at 37°C for 10 minutes, 0.5 ml of 0.5% SDS was added to the reaction solution and the absorbance at 550 nm was then measured. GPC was quantitatively assayed using an aqueous solution of choline with a known concentration for calibration.

### [Example 8] Method of manufacturing PI

40 g of phospholipid derived from soybean was suspended in 400 ml of 10 mM Acetate (pH 5.8) while stirring and then added with 1,400 units of PLB (enzyme fraction A obtained in Example 1) as phospholipid processing agent of the present invention to initiate a reaction at 45°C. After 20 hours, the reaction was terminated and then added with 200 ml of ethanol and 400 ml of hexane, followed by stirring for 1 hour. The hexane layer and the aqueous layer were separated from each other by centrifugal separation, thereby recovering an organic solvent layer. The recovered organic solvent was concentrated under reduced pressure and the concentrate was then added with 150 ml of acetone. The resulting precipitation was collected and dried, thereby obtaining powders with a high concentration of PI (8.2 g). The purity of the obtained phospholipid was measured according to the Standard Oil and Fat Analytical Test Method (established by JAPAN Oil Chemists' Society, 1996). Consequently, the purity was 86.8 mol% with respect to the total phospholipid.

### [Example 9]Method of manufacturing GPC

GPC, GPE, and GP were contained in the aqueous layer obtained by organic solvent extraction from the PLB reactant in the method described in Example 8. 380 ml of the water layer was passed through a column (bed volume: 50 ml) filled with activated carbon, thereby obtaining a colorless passage liquid. The liquid was concentrated under reduced pressure, thereby obtaining 40 ml of a GPC solution. The purity of GPC was 55 wt% from the assay of GPC as described in Example 2.

### [Example 10] Method of manufacturing lysophosphatidylinositol

10 g of PI obtained in Example 8 was added with 20 ml of water and then stirred well, followed by the addition of 100 mg of phospholipase A1 (11,900 U/g) manufactured by Sankyo LifeTech Co., Ltd. An enzyme reaction was then carried out while stirring at 50°C. After 24 hours from the start of the reaction, the reaction solution was treated at 80°C for 30 minutes, thereby terminating the reaction. The reaction solution was dried, added with 100 ml of acetone, and stirred well, followed by obtaining a solid after filtration. The solid was then dried, thereby obtaining 6 g of lysophosphatidylinositol. From the results of a TLC analysis, it was confirmed that the unreacted PI and the free fatty acid of the reaction byproduct had been removed.
TLC carrier: Silica Ge160, layer thickness 2 mm (manufactured by Merck Co., Ltd.), developer: chloroform: methanol: water = 65: 35: 4, coloring method: iodine coloring, Rf value: lysophosphatidylinositol (0.12), PI (0.28), free fatty acid (0.81).

### [Example 11] Method of manufacturing phospholipase B from Penicillium notatum

Phospholipase B was produced from Penicillium notatum as described below based on the well-known method of manufacturing phospholipase B from Penicillium notatum. 100 ml of Penicillium notatum (IFO-4640) preincubated in a Erlenmeyer flask (500 ml in volume) was transferred to 20 liters of a culture medium (3.5% corn steep liquor, 5.5% lactose, 0.7% potassium phosphate, 0.3% magnesium sulfate, 0.5% calcium carbonate, and 0.25% soybean oil, at pH 5.4) sterilized by autoclave and then incubated under aerobic conditions at 26°C for 4 days. After terminating the incubation, fungus bodies were obtained by filtration. The fungus bodies were added with 10 liters of purified water and then homogenized by a homogenizer for 10 minutes to obtain a solubilized enzyme, followed by filtration. Consequently, an eight liter crude enzyme solution was obtained. The filtrate was passed through a column filled with 2 kg of palmitoylated cellulose fiber to adsorb the enzyme and the column was then washed with purified water, followed by eluting the enzyme with a 1 mM phosphate buffer pH 7.0 containing 0.5% Adecatol SO120 and 0.2 mM EDTA. The eluent (10 liters) was loaded on Q-Sepharose ion-exchange column, thereby adsorbing enzyme. The column was washed with a 1 mM phosphate buffer pH 7.0 containing 0.2 mM EDTA, and the enzyme was then eluted with a 10 mM phosphate buffer pH 7.0 containing 0.2 M NaCl and 2 mM EDTA. Subsequently, the enzyme was dialyzed with a 10 mM phosphate buffer pH 7.0 containing 2 mM-EDTA and then desalted and lyophilized, thereby obtaining enzyme powder (3800 U/g).

### [Example 12] Method of manufacturing glyceryl phosphoryl inositol

10 g of PI obtained in Example 8 was added with 100 ml of 2 mM Acetate (pH 4.0) and then stirred well, followed by the addition of 200 mg of phospholipase B obtained from Penicillium notatum as described in Example 11 to carry out an enzyme reaction at 40°C while stirring. After 24 hours from the start of the reaction, the reaction was terminated by an 80°C treatment for 30 minutes. The reaction solution was added with 50 ml of hexane and then stirred, followed by left standing to remove a hexane layer. Then, an aqueous layer was recovered. The aqueous layer was passed through a column previously filled with activated carbon and a passed liquid was then collected, followed by being lyophilized. Consequently, 8-g glyceryl phosphoryl inositol was obtained.

### [Food Production Example 1] Phosphatidylinositol (PI)-containing margarine

PI obtained in Example 8 was added to vegetable oil so as to be 5 wt% of margarine and then stirred with an emulsifier and the like to be homogeneous. The margarine was prepared by the conventional method.

### [Food Production Example 2] Phosphatidylinositol (PI)-containing bread

1 g of PI obtained in Example 8, 15 g of sugar, 2 g of table salt, and 5 g of powdered milk containing fat were dissolved in 70 g of hot water and then added with two chicken eggs and mixed well. The mixture was added to a mixture of 130 g of wheat flour and 2 g of dry yeast, followed by kneading with hands. About 30 g of butter was added to the mixture and the mixture was further kneaded, and then 30 dough balls for bread roll were prepared. Subsequently, the surface of the dough was coated with beaten egg after fermentation. The dough was baked in an oven at 180°C for 15 minutes, thereby obtaining bread rolls.

### [Food Production Example 3] Phosphatidylinositol (PI)-containing udon (Japanese wheat noodles)

2 g of PI obtained in Example 8 was added to 200 g of water together with 20 g of table salt with respect to 400 g of wheat flour, followed by kneading and the mixture was left standing for a sufficient time. Subsequently, the dough was stretched and cut into pieces of about 6 mm in width, thereby obtaining udon.

### [Food Production Example 4] Phosphatidylinositol (PI)-containing beverages

30 g of PI obtained in Example 8 was suspended in a 5-fold volume of olive oil and then heated to 50°C, thereby obtaining an oil phase. 10 g of glycerol fatty acid ester was added as an emulsifier to 90 g of glycerin and then heated to 70°C and dissolved. The solution was gradually added with the above oil phase while stirring. The mixture was emulsified under high pressure using an emulsifying device to obtain an emulsified composition. Subsequently, 180 ml of water was added to 20 g of emulsified composition and stirred, thereby obtaining a PI-containing beverage.

### [Formulation Example 1] Phosphatidylinositol (PI)-containing tablet

| | |
|---|---|
| PI obtained in Example 8 | 120 g |
| Microcrystalline cellulose | 330 g |
| Carmellose-calcium | 15 g |
| Hydroxypropyl cellulose | 10 g |
| Purified water | 60 ml |

The above composition was mixed by the conventional method and then dried, followed by the addition of 10 g of magnesium stearate. The mixture was made into a tablet, thereby obtaining a 100 mg tablet containing PI in an amount of 20 mg per tablet.

### [Formulation Example 2] Phosphatidylinositol (PI)-containing soft capsule

PI obtained in Example 8 was suspended in a 5-fold volume of olive oil and then sufficiently mixed so as to be homogeneous. Subsequently, a capsule was filled up with the mixture using capsule filler, resulting in a capsule with 300 mg content.

### [Cosmetic Production Example] Phosphatidylinositol (PI)-containing cream pharmaceutical (cosmetic)

PI obtained in Example 8 was added to white petrolatum so as to be 10 wt% in amount and then stirred together with aromatic or the like so as to be homogeneous. A cream pharmaceutical was produced by the conventional method.

### [Food Production Example 5] Lysophosphatidylinositol-containing margarine

Lysophosphatidylinositol obtained in Example 10 was added to vegetable oil so as to be 5 wt% of margarine and then stirred with an emulsifier and the like to be homogeneous. The margarine was prepared by the conventional method.

### [Food Production Example 6] Lysophosphatidylinositol-containing bread

1 g of lysophosphatidylinositol obtained in Example 10, 15 g of sugar, 2 g of table salt, and 5 g of powdered milk containing fat were dissolved in 70 g of hot water and then added with two chicken eggs and mixed well. The mixture was added to a mixture of 130 g of wheat flour and 2 g of dry yeast, followed by kneading with hands. About 30 g of butter was added to the mixture and the mixture was further kneaded, and then 30 dough balls for bread roll were prepared. Subsequently, the surface of the dough was coated with a beaten egg after fermentation. The dough was baked in an oven at 180°C for 15 minutes, thereby obtaining bread rolls.

### [Food Production Example 7] Lysophosphatidylinositol-containing udon (Japanese wheat noodles)

2 g of lysophosphatidylinositol obtained in Example 10 was added to 200 g of water together with 20 g of table salt with respect to 400 g of wheat flour, followed by kneading, and then the mixture was left standing for a sufficient time. Subsequently, the dough was stretched and cut into pieces of about 6 mm in width, thereby obtaining udon.

### [Food Production Example 8] Lysophosphatidylinositol-containing beverages

30 g of lysophosphatidylinositol obtained in Example 10 was suspended in a 5-fold volume of olive oil and then heated to 50°C, thereby obtaining an oil phase. 10 g of glycerol fatty acid ester was added as an emulsifier to 90 g of glycerin and then heated to 70°C and dissolved. The solution was gradually added with the above oil phase while stirring. The mixture was emulsified under high pressure using an emulsifying device to obtain an emulsified composition. Subsequently, 180 ml of water was added to 20 g of emulsified composition and stirred, thereby obtaining a lysophosphatidylinositol-containing beverage.

### [Formulation Example 3] Lysophosphatidylinositol-containing tablet

| | |
|---|---|
| Lysophosphatidylinositol obtained in Example 10 | 120 g |
| Microcrystalline cellulose | 330 g |
| Carmellose-calcium | 15 g |
| Hydroxypropyl cellulose | 10 g |
| Purified water | 60 ml |

The above composition was mixed by the conventional method and then dried, followed by the addition of 10 g of magnesium stearate. The mixture was made into a tablet, thereby obtaining a 100 mg tablet containing lysophosphatidylinositol in an amount of 20 mg per tablet.

### [Formulation Example 4] Lysophosphatidylinositol-containing soft capsule

Lysophosphatidylinositol obtained in Example 10 was suspended in a 5-fold volume of olive oil and then sufficiently mixed so as to be homogeneous. Subsequently, a capsule was filled up with the mixture using capsule filler, resulting in a capsule with 300 mg content.

### [Cosmetic Production Example] Lysophosphatidylinositol-containing cream (cosmetic)

Lysophosphatidylinositol obtained in Example 10 was added to white petrolatum so as to be 10 wt% in amount and then stirred together with aromatic or the like so as to be homogeneous. The cream was produced by the conventional method.

### [Food Production Example 9] Glyceryl phosphoryl inositol-containing margarine

Glyceryl phosphoryl inositol obtained in Example 12 was added to vegetable oil so as to be 5 wt% of margarine and then stirred with an emulsifier and the like to be homogeneous. The margarine was prepared by the conventional method.

### [Food Production Example 10] Glyceryl phosphoryl inositol-containing bread

1 g of glyceryl phosphoryl inositol obtained in Example 12, 15 g of sugar, 2 g of table salt, and 5 g of powdered milk containing fat were dissolved in 70 g of hot water and then added with two chicken eggs and mixed well. The mixture was added to a mixture of 130 g of wheat flour and 2 g of dry yeast, followed by kneading with hands. About 30 g of butter was added to the mixture and the mixture was further kneaded, and then 30 dough balls for bread roll were prepared. Subsequently, the surface of the dough was coated with a beaten egg after fermentation. The dough was baked in an oven at 180°C for 15 minutes, thereby obtaining bread rolls.

### [Food Production Example 11] Glyceryl phosphoryl inositol-containing udon (Japanese wheat noodles)

2 g of glyceryl phosphoryl inositol obtained in Example 12 was added to 200 g of water together with 20 g of table salt with respect to 400 g of wheat flour, followed by kneading and then the mixture was left standing for a sufficient time. Subsequently, the dough was stretched and cut into pieces of about 6 mm in width, thereby obtaining udon.

### [Food Production Example 12] Glyceryl phosphoryl inositol-containing beverages

30 g of glyceryl phosphoryl inositol obtained in Example 12 was suspended in a 5-fold volume of olive oil and then heated to 50°C, thereby obtaining an oil phase. 10 g of glycerol fatty acid ester was added as an emulsifier to 90 g of glycerin and then heated to 70°C and dissolved. The solution was gradually added with the above oil phase while stirring. The mixture was emulsified under high pressure using an emulsifying device to obtain an emulsified composition. Subsequently, 180 ml of water was added to 20 g of emulsified composition and stirred, thereby obtaining a glyceryl phosphoryl inositol-containing beverage.

### [Formulation Example 5] Glyceryl phosphoryl inositol-containing tablet

| | |
|---|---|
| Glyceryl phosphoryl inositol obtained in Example 12 | 120 g |
| Microcrystalline cellulose | 330 g |
| Carmellose-calcium | 15 g |
| Hydroxypropyl cellulose | 10 g |
| Purified water | 60 ml |

The above composition was mixed by the conventional method and then dried, followed by the addition of 10 g of magnesium stearate. The mixture was made into a tablet, thereby obtaining a 100 mg tablet containing glyceryl phosphoryl inositol in an amount of 20 mg per tablet.

### [Formulation Example 6] Glyceryl phosphoryl inositol-containing soft capsule

Glyceryl phosphoryl inositol obtained in Example 12 was suspended in a 5-fold volume of olive oil and then sufficiently mixed so as to be homogeneous. Subsequently, a capsule was filled up with the mixture using capsule filler, resulting in a capsule with 300 mg content.

### [Cosmetic Production Example] Glyceryl phosphoryl inositol-containing cream (cosmetic)

Glyceryl phosphoryl inositol obtained in Example 12 was added to white petrolatum so as to be 10 wt% in amount and then stirred together with aromatic or the like so as to be homogeneous. The cream was produced by the conventional method.

### [Industrial Applicability]

The phospholipid processing agent according to the present invention is suitable for the production of functional phospholipids using phospholipids derived from soybean as raw materials.

### [Brief Description of the Drawings]

[Figure 1] Figure 1 illustrates a chromatographic pattern obtained by gel-filtration chromatography of PLB as a phospholipid processing agent according to Example 4 of the present invention.
[Figure 2] Figure 2 illustrates an optimal pH of PLB as a phospholipid processing agent according to Example 5 of the present invention.
[Figure 3] Figure 3 illustrates results of pH stability of PLB as the phospholipid processing agent according to Example 5 of the present invention.
[Figure 4] Figure 4 illustrates results of thermal stability of PLB as the phospholipid processing agent according to Example 5 of the present invention.
[Figure 5] Figure 5 illustrates results of isoelectric focusing of PLB as the phospholipid processing agent according to Example 5 of the present invention.

## Claims

1. A phospholipid processing agent, comprising an enzyme derived from the genus Candida and having a phospholipase B (PLB) activity.

2. A phospholipid processing agent, comprising an enzyme having a phospholipase B (PLB) activity, which substantially does not decompose only phosphatidylinositol in a phospholipid mixture.

3. A phospholipid processing agent according to claim 1 or claim 2, wherein the enzyme having the phospholipase B (PLB) activity further has a lipase activity.

4. A phospholipid processing agent according to any one of claims I to 3, wherein the enzyme having the PLB activity has the following physicochemical properties:
(1) action: an action of hydrolyzing phospholipid into free fatty acid at a molar ratio of two and glyceryl phosphoryl choline at an equimolar ratio;
(2) molecular weight: 53,000±3,000 (determined by SDS electrophoresis);
(3) isoelectric point: pH 4.21±0.2;
(4) optimal pH: about pH 5.5 to 6.5;
(5) pH stability: about pH 5 to 9 (37°C, 90 Minute treatment); and
(6) stability: 55°C (10-minute treatment at pH 5).
(7) substrate specificity: an activity ratio to phosphatidylinositol is 10% or less of that of phosphatidyl choline.

5. A phospholipid processing agent, comprising an enzyme obtained from a culture medium of Candida cylindracea.

6. A phospholipid processing agent, which is obtained by the following steps of:
(1) culturing Candida cylindracea;
(2) condensing a culture medium of Candida cylindracea;
(3) precipitating the enzyme with an organic solvent;
(4) purifying a crude enzyme solution obtained by the step (3) by hydrophobic chromatography; and
(5) isolating and purifying the enzyme obtained by the step (4) by ion-exchange chromatography.

7. A phospholipid processing agent, comprising at lest one enzyme selected from: an enzyme having an amino acid sequence of SEQ ID No. 1; an en enzyme having a PLB activity and 75% or more homology to the amino acid sequence of SEQ ID No. 1; and an enzyme having the PLB activity and having the amino acid sequence of SEQ ID No. 1 with one to several amino acids being deleted, substituted, or added.

8. A phospholipid processing agent, comprising at lest one enzyme selected from: an enzyme having an amino acid sequence of SEQ ID No. 2; an en enzyme having a PLB activity and 75% or more homology to the amino acid sequence of SEQ ID No. 2; and an enzyme having the PLB activity and having the amino acid sequence of SEQ ID No. 2 with one to several amino acids being deleted, substituted, or added.

9. A method of manufacturing phosphatidylinositol and glyceryl phosphoryl choline, comprising allowing the phospholipid processing agent according to any one of claims 1 to 8 to act on a phospholipid mixture.

10. A method of manufacturing phosphatidylinositol, comprising allowing the phospholipid processing agent according to any one of claims 1 to 8 to act on a phospholipid mixture.

11. A method of manufacturing phosphatidylinositol, comprising the steps of:
(1) allowing the phospholipid processing agent according to any one of claims I to 8 to act on a phospholipid mixture;
(2) extracting phosphatidylinositol using an organic solvent; and
(3) precipitating and recovering phosphatidylinositol by a water-soluble or water-miscible alkyl carbonyl alkyl solvent treatment.

12. A method of manufacturing phosphatidylinositol according to any one of claims 9 to 11, wherein the phospholipid mixture is derived from soybean.

13. A phosphatidylinositol, which is obtained by the manufacturing method according to any one of claims 10 to 12, wherein purity of the phosphatidylinositol is 50 mol% or more in total phospholipids.

14. A method of manufacturing glyceryl phosphoryl choline, comprising allowing the phospholipid processing agent according to any one of claims 1 to 8 to act on a phospholipid mixture.

15. A method of manufacturing glyceryl phosphoryl choline, comprising the steps of:
(1) allowing the phospholipid processing agent according to any one of claims 1 to 8 to act on a phospholipid mixture;
(2) extracting and removing a lipid component using a solvent containing an organic solvent and collecting the glyceryl phosphoryl choline in an aqueous layer; and
(3) adsorbing the phospholipid processing agent acting in the step (1) on activated carbon and removing the phospholipid processing agent.

16. A method of manufacturing glyceryl phosphoryl choline according to claims 9, 14, or 15, wherein the phospholipid mixture is derived from soybean.

17. A glyceryl phosphoryl choline, which is obtained by the manufacturing method according to any one of claims 14 to 16, and having a glyceryl phosphoryl choline purity of 55 wt% or more.

18. Use of the phosphatidylinositol according to claim 13 for the production of high-purity glycerophosphoinositol.

19. Use of the glyceryl phosphoryl choline according to claim 17 for the production of high-purity choline phospholipid.

20. Food, medicine, or cosmetic, comprising the phosphatidylinositol manufactured by the manufacturing method according to any one of claims 10 to 12.

21. Food, medicine, or cosmetic, comprising the glyceryl phosphoryl choline manufactured by the manufacturing method according to any one of claims 14 to 16.

22. A lysophosphatidylinositol, which is obtained by allowing an enzyme having a phospholipase A1 or phospholipase A2 activity to act on the phosphatidylinositol manufactured by the manufacturing method according to any one of claims 10 to 12.

23. A method of manufacturing lysophosphatidylinositol, comprising allowing an enzyme having a phospholipase A1 or phospholipase A2 activity to act on the phosphatidylinositol manufactured by the manufacturing method according to any one of claims 10 to 12.

24. Food, medicine, or cosmetic, comprising the lysophosphatidylinositol manufactured by the manufacturing method according to claim 23.

25. A glyceryl phosphoryl inositol obtained by allowing an enzyme having a phospholipase B activity which acts on phosphatidylinositol well to act on the phosphatidylinositol manufactured by the manufacturing method according to any one of claims 10 to 12.

26. A method of manufacturing glyceryl phosphoryl inositol, comprising allowing an enzyme having phospholipase B activity which acts on phosphatidylinositol well to act on the phosphatidylinositol manufactured by the manufacturing method according to any one of claims 10 to 12.

27. Food, medicine, or cosmetic, comprising the glyceryl phosphoryl inositol manufactured by the manufacturing method according to claim 26.

28. Food, medicine, or cosmetic, comprising two or more kinds selected from the group consisting of: the phosphatidylinositol manufactured by the manufacturing method according to any one of claims 10 to 12; the glyceryl phosphoryl choline manufactured by the manufacturing method according to any one of claims 14 to 16; the lysophosphatidylinositol manufactured by the manufacturing method according to claim 23; and the glyceryl phosphoryl inositol manufactured by the manufacturing method according to claim 26.
